# EUROPEAN PATENT SPECIFICATION

(11) **EP 1 286 668 B1**
(45) Date of publication and mention of the grant of the patent: **27.04.2005**
(21) Application number: 00989967.5
(22) Date of filing: 08.12.2000
(51) Int. Cl.: A61K 31/4045, A61K 31/4439, A61P 1/04

(54) **PHARMACEUTICAL COMBINATIONS COMPRISING TEGASEROD UND OMEPRAZOLE AND THEIR USE IN TREATING GASTROINTESTINAL DISORDERS**
PHARMAZEUTISCHE KOMBINATIONEN ENTHALTEND TEGASEROD UND OMEPRAZOLE UND IHRE VERWENDUNG ZUR BEHANDLUNG GASTROINTESTINALER KRANKHEITEN
COMBINAISONS PHARMACEUTIQUES CONTENANTE TEGASEROD ET OMEPRAZOLE ET LEUR UTILISATION DANS LE TRAITEMENT DE TROUBLES GASTRO-INTESTINAUX

(30) Priority: 10.12.1999 US 458388
(43) Date of publication of application: 05.03.2003
(60) Divisional application: 04020247.5
(73) Proprietor: Novartis AG, 4002 Basel (CH); Novartis Pharma GmbH, 1230 Wien (AT)
(72) Inventor: BILLSTEIN, Stephan, Anthony, Franklin Lakes, NJ 07417 (US); DUMOVIC, Peter, Mendham, NJ 07945 (US); FRANCO, Nicola, F-68480 Sondersdorf (FR); IWICKI, Mark, Thomas, Long Valley, NJ 07853 (US); PFANNKUCHE, Hans-Jürgen, 79576 Weil (DE); WILUSZ, Edward, Joseph, Jr., Pittstown, NJ 08867 (US)
(74) Representative: Gros, Florent
(86) International application number: PCT/EP2000/012420
(87) International publication number: WO 2001/041748

(56) References cited:
- EP-A- 0 505 322
- WO-A-93/12785
- WO-A-94/01095
- WO-A-99/17755
- SCOTT & PERRY: "TEGASEROD" DRUGS,AT,ADIS INTERNATIONAL LTD, vol. 58, no. 3, 1999, pages 492-496, XP000874675 ISSN: 0012-6667
- STEADMAN C J ET AL: "Selective 5-Hydroxytryptamine Type 3 Receptor Antagonism With Ondansetron as Treatment for Diarrhea-Predominant Irritable Bowel Syndrome: A Pilot Study" MAYO CLINIC PROCEEDINGS, MAYO MEDICAL VENTURES, ROCHESTER, MN, US, vol. 67, no. 8, August 1992 (1992-08), pages 732-738, XP002098638 ISSN: 0025-6196

## Description

The present invention relates to pharmaceutical combinations comprising tegaserod or pharmaceutically acceptable salt thereof and omeprazole or a pharmaceutically acceptable salt enantiomer or racemale thereof and their uses in treating gastrointestinal disorders.

Serotonin (5-hydroxytryptamine; 5-HT) functions as a neurotransmitter in the mammalian central nervous system (CNS) and in the periphery. Serotonin is one of the transmitters to be recognized for its physiological importance, and agents which interact with 5-HT receptors are currently the focus of much research (P. Bonate, Clinical Neuropharmacology, 1991, Vol. 14(1), pp. 1-16). To-date, the number of serotonin receptor subtypes identified is into the tens, including the major classes, i.e., 5-HT₁, 5-HT₂, 5-HT₃, 5-HT₄, 5-HT₅, 5-HT₆, and 5-HT₇. Because of the multiplicity of serotonin receptor subtypes, the identification of which serotonin receptor subtype is correlated to various physiological/pharmacological actions is complicated.

Serotonin has been known for some years to modulate peristalsis in the gastro-intestinal (GI) tract in various mammalian models. During the mid 1980s, several specific antagonists to the 5-HT₃ receptor subtype were identified and are currently used as anti-emesis/vomiting agents in cancer therapy. 5-HT₃ antagonists have also recently been studied for the treatment of irritable bowel syndrome ("IBS").

A number of gastrointestinal syndromes are related to the production and actions of serotonin, and they have a fairly common occurrence in a very large number of people worldwide. Some of the more well-known gastrointestinal conditions, syndromes or diseases are IBS, gastro-esophageal reflux disease ("GERD") and dyspepsia.

IBS is a chronic condition associated with abdominal pain, bloating and altered bowel function and is estimated to affect as much as 10-20% of the population. Sometimes the disease is referred to as irritable colon, spastic colon, spastic colitis or mucous colitis. The latter two are almost certainly misnomers, as colitis implies inflammation of the colon, and an absence of inflammation is one of the defining observations in a diagnosis of IBS. The cause of IBS is unknown, but a number of factors have been implicated, including diet, lifestyle, depression, anxiety, infections and unrelated inflammatory conditions, including early insult resulting in central neuronal sensitization and sensitizing of neurons in the gut.

Almost all medications currently in use for the treatment of IBS have failed to establish a significant therapeutic effect.

GERD is a condition that is associated with the reflux of gastric contents to the esophagus through the lower esophageal sphincter. GERD is characterized by symptoms of heartburn, bloating, abdominal pain, epigastric pain, early satiety, nausea, regurgitation, burbutence and vomiting. The reflux is thought to occur because of an increased incidence of transient lower esophageal sphincter relaxations allowing gastric contents to enter the esophagus.

Dyspepsia is also an important health problem. The most common conditions that are associated with patients who present with chronic symptoms of dyspepsia are GERD, duodenal ulcer or gastric ulcer and other diagnoses (e.g. functional/non-ulcerative dyspepsia, gallbladder or liver disease).

These conditions or diseases are characterized by altered motility, sensitivity, secretion and/or infections with Helicobacter pylori as well as potentially a psychological (usually subconscious) overlay. At present, only few medications have shown clinically significant efficacy for the treatment of e.g. functional dyspepsia, of which some do exert various adverse effects in man.

Accordingly, there is a need for agents which modulate and normalize altered GI motility, sensitivity and secretion, and Interact with 5-HT receptors involved in various physiological processes and which have broad clinical usefulness for the treatment of the numerous gastrointestinal disorders affecting millions of people each year. More specifically, there is a need for pharmaceutical combinations comprising either 5-HT₄ receptor agonists and a co-agent effective for the treatment of altered gastrointestinal motility, sensitivity and/or secretion and/or abdominal disorders including both functional and organic diseases.

It has now been found that a combination comprising at least one agent interacting with a 5-HT₄ receptor tegaserod, and a co-agent, omeprazole, has a beneficial effect and is useful in the treatment of altered gastrointestinal motility, sensitivity and/or secretion and/or abdominal disorders. This combination may also be used to regulate, stabilize and normalize altered gastrointestinal motility, sensitivity and/or secretion and/or abdominal disorders.

### Definitions

Unless otherwise specified herein, common definitions are intended by the words and terms used herein. For example, the term "pharmaceutical combination" as used herein means a product that results from the mixing or combining of more than one active ingredient and includes both fixed and non-fixed combinations of the active ingredients.

The term "fixed combination" as that term is used herein means that the active ingredients, e.g. tegaserod and a co-agent, are both administered to a patient simultaneously in the form of a single entity or dosage. As an example, a fixed combination would be one capsule containing two active ingredients.

The term "non-fixed combination" as that term is used herein means that the active ingredients, e.g. tegaserod and a co-agent, are both administered to a patient as separate entities either simultaneously, concurrently or sequentially with no specific time limits, wherein such administration provides therapeutically effective levels of the two compounds in the body, preferably at the same time. As an example, a non-fixed combination would be two capsules each containing one active ingredient where the purpose is to have the patient achieve treatment with both active ingredients together in the body.

The term "altered gastrointestinal motility, sensitivity and/or secretion disorder(s)" as used herein includes one or more of the symptoms and conditions which affect the gastrointestinal tract from the mouth to the anus, which include, but are not limited to, heartburn, bloating, postoperative ileus, abdominal pain and discomfort, early satiety, epigastric pain, nausea, vomiting, burbutence, regurgitation, intestinal pseudoobstruction, anal incontinence, GERD, IBS, dyspepsia, chronic constipation or diarrhea, gastroparesis, e.g. diabetic gastroparesis, ulcerative colitis, Crohn's disease, ulcers and the visceral pain associated therewith.

The term "abdominal disorder(s)" as used herein includes those conditions which affect the lower abdomen and include but are not limited to those conditions treated by regulation, stabilization and normalization of enterochromaffin cell functions, GI secretion, motility, afferent and efferent fiber activity and/or abdominal smooth muscle cell activity.

The term "gastro-esophageal reflux disease" and "GERD" as used herein means the incidence of, and the symptoms of, those conditions caused by the reflux of the stomach contents into the esophagus. This includes all forms/manifestations of GERD including, but not limited to, erosive and non-erosive GERD, heartburn and other symptoms associated with GERD.

The term "irritable bowel syndrome" and "IBS" as used herein means a disorder of function involving altered motility, sensitivity and secretion involving primarily the small intestine and large bowel associated with variable degrees of abdominal pain, bloating, constipation or diarrhea without overt bowel inflammation.

The term "dyspepsia" as used herein means a condition characterized by symptoms of epigastric pain, abdominal pain, bloating, early satiety, nausea, heartburn and vomiting as a primary gastrointestinal dysfunction or as a complication due, and not exclusive to disorders such as ulcer disease, appendicitis, gallbladder disturbances, or malnutrition.

The term "gastroparesis" as used herein means a paralysis of the stomach brought about by a motor abnormality in the stomach which is often manifested as delayed gastric emptying. This can also be a complication of diseases such as diabetes, progressive systemic sclerosis, anorexia nervosa, or myotonic dystrophy.

The term "constipation" as used herein means a condition characterized by infrequent and/or difficult evacuation of feces resulting from conditions such as altered GI motility, altered sensation or evacuation functions, altered secretion or reabsorption of electrolytes and water.

The term "diarrhea" as used herein means a condition characterized by frequent evacuations of feces often associated with large volumes and urge resulting from conditions such as altered GI motility, altered sensation and secretion or reabsorption of electrolytes and water.

The term "treat" or "treatment" encompasses the complete range of therapeutically positive effects associated with pharmaceutical medication including reduction of, alleviation of and relief from the symptoms or illness which affect the organism.

### DESCRIPTION

More particularly the present invention provides:
1.1 A pharmaceutical combination comprising:
   a) a first agent which is a compound of formula 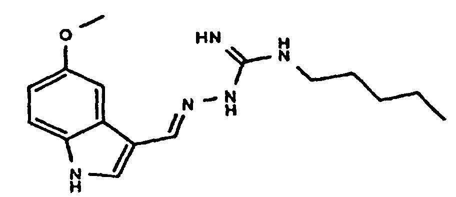 in free form or in pharmaceutically acceptable salt form and a co-agent. This compound has the chemical name of 3-(5-mathoxy-1H-indol-3-yl-methylene)-N-pentylcarbazimidamide, and is also known as tegaserod. It is disclosed as being a 5-HT₄ receptor partial agonist. It may also exist in form of tautomers 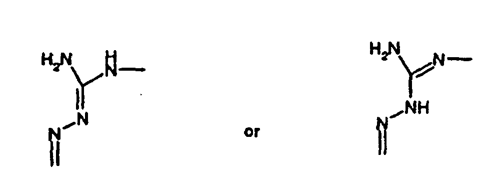 which are included in the present invention. A preferred salt form is the hydrogen maleate. The co-agent in combination is omeprazole marketed under the trade name PRILOSEC® and LOSEC®.

Also included are the pharmaceutically acceptable salts, hydrates, polymorphs, tautomers, racemates, diastereoisomers or enantiomers of all the above-identified first agents or co-agents.

Also within the scope of this invention is the combination of more than two separate active ingredients as set forth above, i.e. a pharmaceutical combination within the scope of this invention could include three active ingredients or more. Further, both the first agent and the co-agent are not the identical active ingredient.

A preferred combination of the invention is one which comprises, as first agent, tegaserod, e.g. in hydrogen maleate salt form, formulated as a solid oral pharmaceutical composition, e.g. a tablet. Representative tegaserod tablets comprise 20 to 60%, e.g. 30 to 50% by weight of a disintegrant; e.g. a super-disintegrant such as crospovidone, based on the total weight of the tablet, e.g. as disclosed in WO 00/10526. An example may be e.g. a tablet comprising 8.31 mg tegaserod in hydrogeno-maleate form (or 6 mg base), 50.00 mg polyplasdone XL, 12.50 mg glyceryl monostearate, 2.50 mg poloxalkol, 37.94 mg lactose, 6.25 mg HPCM, 7.50 mg PEG 4000 and 3.00 mg adsorbed water.

In accordance with another aspect of the invention there is provided a pharmaceutical composition comprising a combination of a first agent and a co-agent as active ingredients, or pharmaceutically acceptable salts, racemates or enantiomers thereof, as defined above under 1.1; in the presence of a pharmaceutically acceptable carrier, and optionally, other therapeutic ingredients. In a preferred embodiment the first agent according to 1.1 is tegaserod in the hydrogen maleate salt form.

It has surprisingly been found that the pharmaceutical combinations and compositions of the present invention provide an enhanced treatment response for altered gastrointestinal motility, sensitivity and/or secretion and/or abdominal disorders mentioned herein. For example, the combination of tegaserod and omeprazole provides not only motility regulation in the upper GI tract but inhibit gastric acid secretion as well which is extremely beneficial for patients suffering from GERD. Furthermore, it has also surprisingly been found that the pharmaceutical combinations and compositions of the present invention provide an enhanced reduction of gastrointestinal pain and other symptoms normally associated with disturbed/altered gastrointestinal motility, sensitivity and/or secretion.

The terms "pharmaceutically acceptable salts" or "a pharmaceutically acceptable salt thereof" refer to salts prepared from pharmaceutically acceptable nontoxic acids or bases including inorganic acids and bases. Suitable pharmaceutically acceptable acid addition salts for the first agent and the co-agents of the present invention include acetic, benzenesulfonic (besylate), benzoic, camphorsulfonic, citric, ethenesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric acid, p-toluenesulfonic, and the like.

To prepare the pharmaceutical compositions of the present invention, the first agent and the co-agent, or their pharmaceutically acceptable salts, racemates or enantiomers are combined in intimate admixture by mixing, blending or combining in any manner known to those of skill in the art, with a pharmaceutically acceptable carrier. The pharmaceutically acceptable carrier may take a wide variety of forms depending on the form of preparation desired for administration.

Any suitable route of administration may be employed for providing a mammal with a therapeutically effective amount of the pharmaceutical combinations and compositions of the present invention. For example, oral, rectal, vaginal, topical, parental (subcutaneous, intramuscular, intravenous, transdermal) and like forms of administration may be employed. Dosage formulations include ointments, foams, gels, transdermal patches, tablets (both fractionable and non-fractionable), caplets, powders for inhalations, gelcaps, capsules, elixirs, syrups, chewable tablets, lozenges, troches, dispersions, aerosols, solutions, fast-dissolving wafers, suppositories or suspensions or other known and effective delivery methods.

In addition to the dosage formulations set out above, the pharmaceutical combinations and compositions of the present invention may also be administered by controlled release means and/or delivery devices such as those described in U.S. Patent Nos. 3,845,770; 3,916,899; 3,536,809; 3,598,123; and 4,008,719 and by "fast-melt" means which include delivery devices which rapidly dissolve in the mouth. Rapid dissolution is meant to include dissolution which takes place in the patient's mouth within less than three minutes. Delivery devices for this type of formulation include, but are not limited to, tablets and capsules. An example of a fast-melt means as used herein is described in U.S. Patent No. 5,178,878 which discloses an effervescent dosage form with microparticles for rapid dissolution of the tablet or capsule.

Oral dosing is preferred. In preparing the compositions in oral dose form, any of the usual pharmaceutical carriers may be employed including any material, composition or vehicle, such as a liquid or solid filler, diluent, excipient, solvent or encapsulating material involved in carrying, formulating or transporting a chemical agent. Specific examples are water, glycols, oils, alcohols and the like in the case of oral liquid preparations. In oral solid forms solid carriers such as starches, sugars, kaolin, lubricants, binders, disintegrating agents and the like may be employed. Oral solid preparations are preferred over the oral liquid preparations. A preferred oral solid preparation is capsules and tablets, because of their ease of administration.

For parental compositions, the carrier will usually comprise sterile water, at least in large part, though other ingredients, to aid solubility for example, may be included. Injectable solutions, for example, may be prepared in which the carrier comprises PEG, saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wetting agent, optionally combined with suitable additives of any nature in minor proportions, which additives do not cause a significant deleterious effect on the skin. It is especially advantageous to formulate the aforementioned pharmaceutical compositions in dosage unit form for ease of administration and uniformity of dosage. Dosage unit form as used herein refers to physically discrete units suitable as unitary dosages, each unit containing a predetermined quantity of active ingredient(s) calculated to produce the desired therapeutic effect in association with the required pharmaceutical carrier.

In another embodiment of the present invention there is provided:
2. A pharmaceutical combination of the first agent and the co-agent; or the pharmaceutically acceptable salts, racemates or enantiomers thereof, or a pharmaceutical composition comprising such a combination in the presence of a pharmaceutically acceptable carrier, as defined above, for use in the treatment of altered gastrointestinal motility, sensitivity and/or secretion and/or abdominal disorders.
3. Use of a pharmaceutical combination of the first agent and the co-agent, or the pharmaceutically acceptable salts, racemates or enantiomers thereof, as defined above, in the manufacture of a medicament for use in the treatment of altered gastrointestinal motility, sensitivity and/or secretion and/or abdominal disorders.
4. Use of the first agent and the co-agent, or the pharmaceutically acceptable salts, racemates or enantiomers thereof, as defined above, In the manufacture of a pharmaceutical composition for use in the treatment of altered gastrointestinal motility, sensitivity and/or secretion and/or abdominal disorders.

Generally, the pharmaceutical combinations or compositions of the present invention are employed for the treatment of altered gastrointestinal motility, sensitivity and/or secretion and/or abdominal disorders including, but not limited to, heartburn, bloating, postoperative ileus, abdominal pain and discomfort, early satiety, epigastric pain, nausea, vomitting, burbulence, regurgitation, Intestinal pseudoobstruction, anal incontinence, GERD, IBS, dyspepsia, chronic constipation or diarrhea, gastroparesis, e.g. diabetic gastroparesis, ulcerative colitis, Crohn's disease, ulcers or the visceral pain associated therewith. In addition, the pharmaceutical combinations and compositions of the invention may also be employed as laxatives, as a preparation for a patient for colonoscopy, or as a means of regulating, stabilizing or normalizing gastrointestinal disorders, through for example, regulation, stabilization or normalization of enterochromaffin cell functions, GI secretion, afferent and efferent fiber activity or abdominal smooth muscle cell activity. The pharmaceutical combinations and compositions of the invention may also be useful in the treatment of menstrual cramps or spastic or interstitial cystitis.

More specifically, the first agent as described above may be combined according to the invention with the co-agent to treat GERD, dyspepsia, IBS, visceral pain and other abdominal disorders;

The therapeutically effective dosage of the pharmaceutical compositions of this invention will vary with the severity of the condition to be treated, and the route of administration. The dose, and perhaps the dose frequency, will also vary according to the age, body weight, and response of the individual patient. In general, the combination of the first agent and the co-agent may be administered in a molar ratio having a range of from about 0.01 to about 2 for the first agent to a range of from about 0.01 to 1000 for the co-agent. As an example, the molar ratio for the first agent to the co-agent is about 1:1000 (first agent to co-agent). As a more specific example, the molar ratio for the first agent to the co-agent may be about 1:1000, 1:500, 1:200, 1:100, 1:20, 1:5, 1:1 or 1:0.01. A preferable molar ratio is about 1:20, even more preferably about 1:5 and most preferably about 1:1.

The total daily dose range, which comprises the above-described molar ratio, for the conditions described herein, may be administered in a range of from about 0.01 mg to about 1000 mg. The daily dose range may be about 800 mg, 600 mg, 400 mg, 200 mg, 100 mg, 50 mg, 20 mg, 10 mg, 5 mg, 1 mg, 0.1 mg or 0.01 mg. Preferably, a daily dose range should be between about 0.5 mg to about 100 mg, while most preferably, a daily dose range should be between about 5 mg to about 75 mg. It is preferred that the doses are administered OD (once daily) or BID (2 times a day). In managing the patient, the therapy should be initiated at a lower dose, perhaps about 5 mg to about 10 mg, and increased up to about 50 mg or higher depending on the patient's response. It may be necessary to use dosages outside these ranges in some cases as will be apparent to those skilled in the art. Further, it is noted that the clinician or treating physician will know how and when to interrupt, adjust, or terminate therapy in conjunction with individual patient response. The term "therapeutically effective amount" is encompassed by the above-described molar ratio and dosage amounts and dose frequency schedule. A "therapeutically effective amount" can be administered in both a fixed or non-fixed combination of a first agent, e.g. tegaserod, and the co-agent.

### EXAMPLES

The present invention is further described by the following example.

### Example 1

### Treatment of non-erosive GERD with the combination of tegaserod and omeprazole

Patients selected for the study are patients with heartburn, the target symptom in patients with non-erosive GERD, as the predominant upper gastrointestinal symptom during the last three (3) months prior to entry into the study and with a history of episodes of heartburn occurring on at least 3 days/week. Patients with GERD and without endoscopic signs of erosive esophagitis are included In the study. Among other factors, patients who are treated with histamine H₂-receptor antagonists (H₂RAs) In prescription doses or PPIs within one month prior to entry Into the baseline phase of the study (Day -14) and patients who need continuous use of PPIs within three months prior to entry into the baseline phase of the study are excluded.

The study consists of a one-week screening period and a 2-week drug-free baseline period, followed by an 8-week, double-blind, placebo-controlled treatment period. During the screening period (Day -21 to Day -14), an endoscopy is performed to rule out the presence of erosive esophagitis. During baseline (Day -14 to Day 1), the patient's symptoms of GERD are documented In a daily diary. At the start of the period, medications for GERD such as H₂RAs, PPIs, prokinetics and other disallowed medication are withdrawn and the patients are instructed not to change their diet or lifestyle during the trial. Patients are allowed to take Maalox tablets as a rescue medication for the control of their symptoms. Patients entering the double-blind period have episodes of heartburn on three (3) or more days the last week of the baseline period.

Patients are randomized in equal groups during the double-blind, placebo-controlled period of the study. This period of the study lasts eight (8) weeks and there are 12 treatment arms. The patients in each group receive one of the following regimens: 1) placebo, 2) tegaserod 0.4 mg/day, 3) tegaserod 1 mg/day, 4) tegaserod 4 mg/day, 5) ranitidine 300 mg/day, 6) omeprazole 20 mg/day, 7) tegaserod 0.4 mg/day plus ranitidine 300 mg/day, 8) tegaserod 1 mg/day plus ranitidine 300 mg/day, 9) tegaserod 4 mg/day plus ranitidine 300 mg/day, 10) tegaserod 0.4 mg/day plus omeprazole 20 mg/day, 11) tegaserod 1 mg/day plus omeprazole 20 mg/day and 12) tegaserod 4 mg/day plus omeprazole 20 mg/day, given orally bid for the 8 weeks. The administration as per the twelve (12) groups above is within thirty (30) minutes before meal time In the morning and in the evening. During the 8 weeks, patients continue to complete the daily diary and use only Maalox tablets as rescue medication for the control of their symptoms.
The combination of 1) tegaserod with omeprazole and 2) tegaserod with ranitidine significantly reduces the episodes of heartburn occurring per week during the 8 week period of the double-blind, placebo-controlled period of the study as compared to any of placebo, tegaserod, ranitidine and omeprazole alone. The tegaserod combinations also reduces the other symptoms of GERD including, abdominal pain, bloating and regurgitation. Further, patients show a significant improvement in quality of life factors as compared to any of placebo, tegaserod, ranitidine and omeprazole alone.

A pharmaceutical combination according to the invention, tegaserod, and omeprazole, may also be tested clinically, e.g. using a methodology as disclosed by Talley NJ, et al., in Gastroenterol. Intl., 1993, 6(4), 189:211, or by Veldhuyzen van Zanten SJO et al., in Gut 1999, 45 (Suppl.II), 1169:1177. The dosage is preferably oral and administration may be preferably once or twice a day.

## Claims

1. A pharmaceutical combination comprising
a. tegaserod or a pharmaceutically acceptable salt thereof; and
b. omeprazole or a pharmaceutically acceptable salt, enantiomer or racemate thereof.

2. The use of
a. tegaserod or a pharmaceutically acceptable salt thereof; and
b. omeprazole or a pharmaceutically acceptable salt, enantiomer or racemate thereof
in the manufacture of a medicament.

3. The use of
a. tegaserod or a pharmaceutically acceptable salt thereof; and
b. omeprazole or a pharmaceutically acceptable salt, enantiomer or racemate thereof
in the manufacture of a medicament for treating GERD.

4. A pharmaceutical composition for the treatment of GERD comprising
a. tegaserod or a pharmaceutically acceptable salt thereof;
b. omeprazole or a pharmaceutically acceptable salt, enantiomer or racemate thereof; and
c. pharmaceutically acceptable carriers.

5. The composition of claim 4, wherein said composition is to be administered once or twice daily in an oral dosage form.

6. The composition of claim 4, or claim 5, wherein said composition is to be administered in a fast-melt dosage form.

## Patentansprüche

1. Pharmazeutische Kombination, umfassend
a. Tegaserod oder ein pharmazeutisch annehmbares Salz davon und
b. Omeprazol oder ein pharmazeutisch annehmbares Salz, Enantiomer oder Racemat davon.

2. Verwendung von
a. Tegaserod oder einem pharmazeutisch annehmbaren Salz davon und
b. Omeprazol oder einem pharmazeutisch annehmbaren Salz, Enantiomer oder Racemat davon
bei der Herstellung eines Arzneimittels.

3. Verwendung von
a. Tegaserod oder einem pharmazeutisch annehmbaren Salz davon und
b. Omeprazol oder einem pharmazeutisch annehmbaren Salz, Enantiomer oder Racemat davon
bei der Herstellung eines Arzneimittels zur Behandlung der gastro-ösophagealen Refluxkrantcheit (GERD = Gastro-esophageal reflux disease).

4. Pharmazeutische Zusammensetzung für die Behandlung von GERD, umfassend
a. Tegaserod oder ein pharmazeutisch annehmbares Salz davon,
b. Omeprazol oder ein pharmazeutisch annehmbares Salz, Enantiomer oder Racemat davon und
c. pharmazeutisch annehmbare Träger.

5. Zusammensetzung nach Anspruch 4, wobei die Zusammensetzung einmal oder zweimal täglich in einer oralen Dosierungsform über eine Sonde verabreicht wird.

6. Zusammensetzung nach Anspruch 4 oder Anspruch 5, wobei die Zusammensetzung in einer Lingual(Fast-melt)-Dosierungsform über eine Sonde verabreicht wird.

## Revendications

1. Combinaison pharmaceutique comprenant
a. le tégaserod ou un de ses sels pharmaceutiquement acceptables ; et
b. l'oméprazole ou un sel pharmaceutiquement acceptable, énantiomère ou racémate de ce composé.

2. Utilisation
a. du tégaserod ou d'un de ses sels pharmaceutiquement acceptables ; et
b. de l'oméprazole ou d'un sel pharmaceutiquement acceptable, énantiomère ou racémate de ce composé
dans la fabrication d'un médicament.

3. Utilisation
a. du tégaserod ou d'un de ses sels pharmaceutiquement acceptables ; et
b. de l'oméprazole ou d'un sel pharmaceutiquement acceptable, énantiomère ou racémate de ce composé
dans la fabrication d'un médicament pour le traitement de la GERD.

4. Composition pharmaceutique pour le traitement de la GERD comprenant
a. le tégaserod ou un de ses sels pharmaceutiquement acceptables ;
b. l'oméprazole ou un sel pharmaceutiquement acceptable, énantiomère ou racémate de ce composé ; et
c. des véhicules pharmaceutiquement acceptables.

5. Composition selon la revendication 4, **caractérisée en ce que** ladite composition est administrée par tube une ou deux fois par jour sous une forme de dosage oral.

6. Composition selon la revendication 4 ou 5, **caractérisée en ce que** ladite composition est administrée par tube sous une forme de dosage rapidement mélangée.
